# EUROPEAN PATENT APPLICATION

(11) **EP 2 462 928 A1**
(43) Date of publication of application: **13.06.2012**
(21) Application number: 11009696.3
(22) Date of filing: 08.12.2011
(51) Int. Cl.: A61K 9/70, A61K 9/48, A61K 39/36, A61P 37/08

(54) **Sheet-form preparation and method for producing the same**

(30) Priority: 10.12.2010 JP 2010276190
(71) Applicant: Nitto Denko Corporation, Osaka 567-8680 (JP)
(72) Inventor: Daisuke, Asari, Ibaraki-shi Osaka 567-8680 (JP); Takuya, Shishido, Ibaraki-shi Osaka 567-8680 (JP); Mitsuhiko, Hori, Ibaraki-shi Osaka 567-8680 (JP); Toshihiko, Okazaki, Ibaraki-shi Osaka 567-8680 (JP)
(74) Representative: Müller-Boré & Partner Patentanwälte

(57) **Abstract**

The present invention provides a sheet-form preparation that can be easily dissolved intraorally, allows the dissolution time thereof to be easily controlled, and can stably contain a drug except an allergenic protein from cedar pollen. The sheet-form preparation contains water, gelatin, and a drug except an allergenic protein from cedar pollen.

## Description

The present invention relates to a sheet-form jelly-like preparation that contains a drug except an allergenic protein from cedar pollen and that dissolves intraorally; and a method for producing the sheet-form preparation.

Orally available drugs in the current market are in the forms of uncoated tablets, coated tablets, capsules, powders, granules, liquid medicines, and the like. Further, intraorally disintegrating tablets and fast dissolving intraoral films are in the market as drugs disintegrated in the mouth cavity and absorbed at the digestive tract.

Recently, one dosage form is focused on in which a drug is taken by disintegrating or dissolving the drug only with saliva and without intraorally chewing the drug; such a dosage form improves benefits of patients and caregivers. This is due to an increase in the number of patients with disability in ingestion of food and drink, in other words, those having difficulty in mastication and swallowing, involving an increase in the old people population. In addition, the Silver Science Kenkyu Shouwa 62 nendo Kenkyu Houkoku (Silver Science research report) of the former Ministry of Welfare (the present Ministry of Health, Labor and Welfare) named "Koreisha ni toyosaiteki na shinkiseizai oyobi shinkihosoyoki no sakuseikenkyu" (Research of producing an optimal new preparation and packaging container for medicating elderly people), 1988, Masayasu SUGIHARA et al. reported that semisolid formulations (e.g. jelly, yogurt, and pudding) are the expected dosage form of drugs in the future.

The aforementioned backgrounds urge recent development of jelly-like drug-containing formulations, and some kinds of products have been already in the market in Japan.
All of these jelly-like formulations, however, are of portion-packaged type taken with a spoon or the like tools, or of pillow-packaged type taken by pushing it out from the package. Further, the jelly itself is not intraorally dissolved although it is easily dispersed by physical force upon swallowing.

Examples of the water-containing jelly-like dosage form disclosed so far include jelly formulations containing carrageenan, locust bean gum, and polyacrylic acid or its partially neutralized product or its salt (see Patent Document 1), and pharmaceutical jelly composition containing a jelly base and an alkaline salt (see Patent Document 2).
These jelly formulations, however, are those containing a gelling agent thermoreversible at high temperatures (about 60°C to 100°C) or those containing an irreversible gelling agent which is prepared by cross-linking a gelling agent; that is, the jelly itself is not intraorally dissolved but easily dispersed by physical force upon swallowing.
Therefore, these conventional jelly formulations require heating at high temperatures upon preparation or contain a metal salt as a cross-linking agent, so that poor stability thereof may be a problem particularly in the case that the formulations contain drugs having poor heat stability or proteins or peptides strongly interacting with metal salts.

Further, as is disclosed in Patent Document 3 and Patent Document 4, formulations in film shape are also known in which a drug is dispersed or dissolved in a water-soluble polymer.
Such conventional formulations in film shape, however, require a certain quantity of saliva for intraorally dissolving or swelling them using water-soluble polymers. Thus, some dysphagia patients may take a long period to dissolve the formulations. Further, the film-form preparations easily absorb water; thus, disadvantageously, they easily stick to the oral mucous membrane and cause discomfort. Particularly in the case of intraorally soluble film-form preparations, the solubility and the thickness and size of the film are correlative to each other. As a result, it is difficult to prepare a film containing a drug as much as more than 100 mg.
With respect to the production method thereof, the Patent Documents disclose that such formulations in film shape may be prepared by dissolving a water-soluble polymer in water as a solvent, dissolving a drug thereinto, and heat-drying it. Particularly in the case of drugs that are vulnerable to heat, however, a decrease in the drug content due to heat is a concern. If the drug is in a liquid form, the sheet-form formulation may be disadvantageously dissolved, so that keeping of a given shape may be difficult.

Patent Document 1: JP-A 09-187233
Patent Document 2: JP-A 2004-99558
Patent Document 3: JP-T 2005-511522
Patent Document 4: JP-T 2009-507854

In view of the current situation described above, the present invention aims to provide a sheet-form preparation that can be easily dissolved intraorally, that can allow the dissolution time thereof to be easily controlled, and that can stably contain a drug except an allergenic protein from cedar pollen; and a method for producing the sheet-form preparation.

As a result of earnest studies in order to solve the problems described above, the present inventors have discovered that a sheet-form preparation having physical properties without problems in use can be prepared if the preparation contains, as the base material, gelatin that gels at ordinary temperature and maintains an individual state, that easily dissolves at a temperature close to the body temperature, and that contributes to the stabilization of a drug that is vulnerable to heat, and contains, if necessary, sugars, sugar alcohols, polyethylene glycol, crystalline cellulose, and the like. They have also found that such a sheet-form preparation has preparation characteristics suitable for intraoral drug administration including sublingual administration. Thereby, the present inventors have completed the present invention.

That is, the present invention relates to a sheet-form preparation comprising: water; gelatin; and a drug except an allergenic protein from cedar pollen.
The sheet-form preparation of the present invention preferably further comprises at least one selected from the group consisting of sugars, sugar alcohols, and sugar fatty acids.
The sheet-form preparation of the present invention preferably further comprises polyethylene glycol or a derivative thereof, and also preferably further comprises crystalline cellulose.
The drug except an allergenic protein from cedar pollen is preferably provided in a liquid or solid form containing this drug.
The amount of the gelatin is preferably 2 to 40 wt% based on the total weight.
The sheet-form preparation of the present invention preferably has a thickness within the range of 30 to 5000 µm, and also preferably has a planar surface area within the range of 0.5 to 6.0 cm².
The present invention also relates to a method for producing the sheet-form preparation of the present invention. The method comprises the steps of: preparing a mixed solution by mixing water, gelatin, and a drug except an allergenic protein from cedar pollen; and forming a thin film using the mixed solution. In the method, a water content in the obtained sheet-form preparation is adjusted by adjusting the amount of water to be added in the step of preparing a mixed solution or by drying the thin film after the step of forming a thin film.
Hereinafter, the present invention will be described in detail.

The sheet-form preparation of the present invention contains water, gelatin, and a drug except an allergenic protein from cedar pollen.
The sheet-form preparation of the present invention having such a composition is used adequately for intraoral hyposensitization therapy, which requires control of sensitized time, and is particularly suited for sublingual hyposensitization therapy. In addition, when the sheet-form preparation of the present invention contains gelatin and a specific stabilizing agent, the drug except an allergenic protein from cedar pollen, especially proteins and peptides, can be stably maintained.

The thickness of the sheet-form preparation of the present invention is not particularly limited, and it is preferably 30 to 5,000 µm. A film having a thickness of less than 30 µm may suffer problems in sheet strength and product handleability, while a film having a thickness of higher than 5,000 µm may cause discomfort when intraorally, in particular, sublingually, administered.
In addition, the size of the sheet-form preparation of the present invention is not particularly limited, and it is preferable that the planar surface area is within the range of 0.5 to 6.0 cm². A film having a surface area of less than 0.5 cm² may cause difficulty in handling when pinch-administering the sheet-form preparation, while a film having a surface area of higher than 6.0 cm² may not be perfectly inserted intraorally, in particular, sublingually.
In addition, the planar shape of the sheet-form preparation of the present invention is not particularly limited. Examples thereof include rectangles such as oblongs and squares, polygons such as pentagons, circles, ellipses, and the like. Polygons referred to herein include, in addition to complete polygons, shapes having a slight curve in an angular portion.
The term "sheet-form" herein is a concept that includes "film-form".

The sheet-form preparation of the present invention contains gelatin.
The gelatin is an ingredient that constitutes the base material of the sheet-form preparation of the present invention, and has a film-forming ability and edibility.
By containing such a gelatin, the sheet-form preparation of the present invention gels at ordinary temperature and can be dissolved easily at a temperature close to the intraoral body temperature. Further, gelatin can gel at the lowest temperatures among thermoreversible gelling agents. Thus, preparations can be produced at around ordinary temperature to 40°C, so that the stability of drugs having low heat stability can be maintained during production.
The term "edibility" herein means that the gelatin is orally administrable and is pharmaceutically acceptable.

The gelatin is preferably in a grade referred to as water-soluble gelatin which is soluble in water at ordinary temperature. Using the water-soluble gelatin enables to prepare the sheet-form preparation of the present invention at near ordinary temperature and to secure stability of the drug described below during production.
The term "water-soluble gelatin" herein indicates a gelatin such that 1 g of gelatin dissolves in 20 mL of water at ordinary temperature (30°C).

Further, the gelatin preferably has a characteristic such that it does not gel at 32°C but gels at 5°C when turned into an aqueous solution at a concentration of 10 wt%. This is because some gelatins having such a characteristic may sufficiently provide the effects of the present invention depending on the molecular weight thereof and the amount of hydroxyproline therein even if they are beyond the grade of water-soluble gelatins.

Examples of the gelatin used in the sheet-form preparation of the present invention include those prepared by enzymatically decomposing and extracting proteins contained in animal skin or bone, such as those obtained by acid-treating or alkali-treating proteins derived from pigs, cattle, and fish.
The above-mentioned gelatin is particularly preferably a gelatin derived from fish or pigs from the viewpoints of its producibility at ordinary temperature during production and stability of the drug which is vulnerable to heat during production.
From such viewpoints, any gelatin may be adequate as long as it contains hydroxyproline in the amino acid composition in an amount of 5.2 to 9.2 mol% and it has an average molecular weight of exceeding 90,000. Examples of such a gelatin include those derived from fishes such as a salmon-derived gelatin (hydroxyproline amount in amino acid composition: 5.4 mol%), carp-derived gelatin (hydroxyproline amount in amino acid composition: 7.6 mol%), and tilapia-derived gelatin (hydroxyproline amount in amino acid composition: 8.0 mol%). Particularly preferable is a tilapia-derived gelatin.

Here, the above-mentioned amino acid composition is obtained by analysis wherein gelatin is hydrolyzed, then separated by ion-exchange chromatography, and detected by ninhydrin.
Specific examples of the amount of hydroxyproline in the amino acid composition (mol%) obtained by the above-described method are as follows.
Fowl: 10.8 mol%
Ostrich: 10.4 mol%
Mouse: 8.7 mol%
Pig: 9.4 mol%
Cattle: 9.5 mol%

In addition, any gelatin may be preferable regardless of the amount of hydroxyproline in the amino acid composition as long as it has an average molecular weight of 50,000 to 90,000.
The term "average molecular weight" herein means a weight average molecular weight measured by gel-filtration chromatography analysis.
Further, the average molecular weight herein means a molecular weight of each polypeptide chain monomer, not the molecular weight of the polypeptide chain trimer, of gelatin.

In the sheet-form preparation of the present invention, the amount of the gelatin is preferably 2 to 40 wt%, and more preferably 3 to 30 wt%, based on the total weight of the sheet-form preparation of the present invention. If the amount is less than 2 wt%, the gelatin may not gel at ordinary temperature. On the other hand, if the amount is in excess of 40 wt%, the intraoral solubility of the sheet-form preparation of the present invention is extremely low, which may cause a problem during use.

In addition to the above-mentioned gelatin which is an edible polymer, the sheet-form preparation of the present invention may also contain a suitable amount of an edible polymer that is soluble only in water or an edible polymer that dissolves neither in water nor in an organic solvent (hereafter, these are also collectively referred to as other edible polymers) in combination, to the extent that they do not inhibit the effects of the present invention.

Examples of the other edible polymers include synthetic macromolecular compounds such as polyethylene glycol, polyvinyl alcohol, carboxyvinyl polymer, hydroxypropylmethyl cellulose, hydroxyethyl cellulose, methyl cellulose, ethyl cellulose, hydroxypropyl cellulose with a low substitution degree, crystalline cellulose, sodium carboxymethyl cellulose, calcium carboxymethyl cellulose, carboxymethyl cellulose, and sodium carboxymethyl starch; and macromolecular compounds obtained from natural products such as dextran, casein, guar gum, xanthan gum, tragacanth gum, acacia gum, gum arabic, gellan gum, and starch. Each of these other edible polymers can be used alone or two or more of these can be used in combination.
The amount of the other edible polymers is preferably 0.1 to 10 wt% based on the total weight of the sheet-form preparation of the present invention.

The sheet-form preparation of the present invention contains a drug except an allergenic protein from cedar pollen.
The drug except an allergenic protein from cedar pollen is not particularly limited, and is preferably a drug which can be administered to mammals, such as human, sublingually, intraorally, or through the intestinal tract, that is, can be orally administered. Specific examples of such a drug include general anesthetics, sedative hypnotics, antiepileptic drugs, antipyretic-analgesic-antiinflammatory drugs, anti-vertiginous drugs, psychoneurotic drugs, central-nervous-system drugs, antidementia drugs, local anesthetics, skeletal muscle relaxants, autonomic-nervous-system drugs, antispasmodics, antiparkinson drugs, antihistamines, cardiotonics, antiarrhythmic drugs, diuretics, hypotensive agents, vasoconstrictors, coronary vasodilators, peripheral vasodilators, antiarteriosclerotic drugs, circulatory-system drugs, respiratory stimulants, cough suppressants and expectorants, hormone drugs, external preparations for purulent diseases, analgesic-antipruritic-astringent-antiphlogistic drugs, drugs for parasitic skin diseases, hemostatics, gout remedies, antidiabetic drugs, antineoplastics, antibiotics, chemotherapeutic drugs, narcotic drugs, smoking cessation aids, and vaccines.
The above drugs each may be used in an amount enough to cause desired results, such as therapeutic results, through treatment for diseases, conditions, or disorders, that is, in an amount also called an effective dose herein. The effective dose of a drug means that the drug is used in an amount which does not cause toxicity but is sufficient to show the selected effect for a specific period, for example. Such an amount can be easily determined by the skilled person of the technical field of the present invention based on the conventional techniques.
Further, the allergenic protein from cedar pollen is typically included in a cedar pollen extract. In one embodiment of the present invention, the drug can exclude the cedar pollen extract.

Further, the drug except an allergenic protein from cedar pollen may be a solid drug or a liquid drug. The solid drug herein means a drug which is in a solid state at room temperature (25°C), in other words, a drug having a melting point of higher than 25°C. The melting point herein is a value determined using a DSC, type DSC6220 (Seiko Instruments Inc. (SII)).

Further, the liquid drug except an allergenic protein from cedar pollen is a drug having fluidity at room temperature, or 25°C, in other words, having a viscosity of 0.05 to 100,000 mPa·s. The viscosity of the drug is measured using an E-type viscometer while the drug is kept at 25°C.

The amount of the drug except an allergenic protein from cedar pollen depends on the properties and the like thereof. In general, the amount is preferably 1 x 10⁻¹⁰ to 80 wt% to the total weight of the sheet-form preparation of the present invention. If the amount is less than 1 x 10⁻¹⁰ wt%, many drugs may not show their efficacy from the viewpoint of clinical effects. If the amount is in excess of 80 wt%, the strength of the sheet-form preparation of the present invention may be reduced noticeably, likely causing problems in the ability to maintain the shape. The amount of the drug except an allergenic protein from cedar pollen is more preferably in the range of 1 x 10⁻⁶ to 50 wt%. An amount in this range probably leads to preparation of preparations without problems during production and in practice.

The sheet-form preparation of the present invention contains water.
The water is an ingredient that has an effect of assisting dissolution of the sheet-form preparation.
In addition, controlling of the water content in the sheet-form preparation of the present invention enables to easily control the dissolution time of the sheet-form preparation. Consequently, the sheet-form preparation of the present invention is appropriate for both cases of intraorally dissolving and taking the drug or of intraorally, in particular, sublingually, slowly dissolving and control-releasing the drug.
In the present invention, the water content is preferably 1 to 60 wt%, and more preferably 5 to 50 wt%, based on the total weight of the sheet-form preparation. If the water content is less than 1 wt%, the intraorally solubility may be extremely poor, which may cause a problem during use. On the other hand, if the water content is in excess of 60 wt%, the storage stability at ordinary temperature regarding physical properties may be poor.

The sheet-form preparation of the present invention preferably further comprises an additive which improves physical properties and solubility, such as at least one selected from the group consisting of sugars, sugar alcohols, and sugar fatty acids.
Examples of the sugars include monosaccharides, disaccharides, and tri- to hexa-saccharides as indicated below.
Examples of the monosaccharides include aldotetroses such as erythrose and threose, aldopentoses such as ribose, lyxose, xylose, and arabinose, aldohexoses such as allose, talose, gulose, glucose, altrose, mannose, galactose, and idose, ketotetroses such as erythrulose, ketopentoses such as xylulose and ribulose, ketohexoses such as psicose, fructose, sorbose, and tagatose. Examples of the disaccharides include α-diglucosides such as trehalose, kojibiose, nigerose, maltose, and isomaltose, β-diglucosides such as isotrehalose, sophorose, laminaribiose, cellobiose, and genthiobiose, and α,β-diglucosides such as neotrehalose, as well as lactose, sucrose, and isomaltulose (Palatinose). Examples of the trisaccharides include raffinose. Examples of the tri- to hexa-saccharide oligosaccharides include fructooligosaccharide, galactooligosaccharide, xylooligosaccharide, isomaltooligosaccharide, chitin oligosaccharide, chitosan oligosaccharide, oligoglucosamine, dextrin, and cyclic oligosaccharides such as cyclodextrin.

Examples of alcohols of the monosaccharides include tetritols such as erythritol, D-threitol, and L-threitol, pentitols such as D-arabinitol and xylitol, hexitols such as D-iditol, galactitol (dulcitol), D-glucitol (sorbitol), and mannitol, and cyclitols such as inositol. Examples of alcohols of the disaccharides include maltitol, lactitol, and reduced palatinose (isomalt). Examples of oligosaccharides include pentaerythritol and reduced maltose syrup.
In the sheet-form preparation of the present invention, the sugars or sugar alcohols may be substituted. Further, each of these may be used alone or two or more of these may be used in combination.

In order to easily intraorally dissolve the sheet-form preparation of the present invention and not to greatly vary the viscosity of the solution in the production process, the sugars or sugar alcohols are preferably mono- to tri-saccharides or sugar alcohols thereof.

Examples of the sugar fatty acids include sorbitan fatty acid esters and sucrose fatty acid esters.
Examples of the sorbitan fatty acid esters include sorbitan monooleate, sorbitan trioleate, sorbitan sesquioleate, sorbitan cocoate, and polyoxyethylene sorbitan fatty acid esters.
Examples of the sucrose fatty acid esters include sucrose stearate, sucrose oleate, sucrose palmitate, sucrose myristate, sucrose behenate, sucrose erucate, and sucrose-mixed fatty acid esters.
These sugar fatty acids are useful as an antifoaming agent, as well as stabilizing agents for proteins and peptides; thus, they are very convenient.

Further, the sheet-form preparation of the present invention preferably contains polyethylene glycol or a derivative thereof and cellulose as additives for improving the physical properties.
The polyethylene glycol preferably has an average molecular weight of 200 to 20,000, and more preferably an average molecular weight of 400 to 8,000. If the average molecular weight is 200 or lower, the additives may have high plasticity, so that sufficient physical properties required for use may not be achieved. If the average molecular weight is in excess of 20,000, the preparation may have high viscosity upon dissolution, so that it may cause discomfort in the mouth cavity. The average molecular weight herein is determined by the average molecular weight test described in the item of Macrogol 400 in the official monographs of the Japanese Pharmacopoeia 15th edition.

The cellulose is preferably crystalline cellulose or powder cellulose, and more preferably crystalline cellulose.
The cellulose preferably has an average particle size of 0.01 to 100 µm. It more preferably has an average particle size of 0.01 to 50 µm. If the average particle size is smaller than 0.01 µm, the particles are likely to aggregate in the solution during preparation, and they may adversely deteriorate the physical properties. If the average particle size is larger than 100 µm, the particles are likely to precipitate in the solution during preparation and they may cause sense of residues and discomfort when the preparation is intraorally administered. The average particle size herein is a 50% average particle size determined using a laser scattering particle size distribution measurement device.

In the sheet-form preparation of the present invention, the amount of the additives is preferably 1 to 80 wt%, and more preferably 5 to 70 wt%, based on the total weight of the sheet-form preparation of the present invention. If the amount is less than 1 wt%, the physical properties may not be sufficiently maintained during use. On the other hand, if the amount is in excess of 80 wt%, it may be difficult to control the physical properties of the sheet-form preparation by the added additives.

If necessary, the sheet-form preparation of the present invention may appropriately contain, as ingredients constituting the base material, a perfume, a flavoring substance, a sweetening agent, a colorant, an antiseptic, an antioxidant, a stabilizing agent, a surfactant, and the like, in addition to the ingredients mentioned above. These ingredients are not particularly limited and conventionally known ones may be used.

Since the sheet-form preparation of the present invention contains gelatin as mentioned above, it gels at ordinary temperature and can be easily intraorally dissolved at a temperature close to the body temperature. Further, if the formulation contains gelatin and a specific additive, the physical properties during use can be significantly improved. Furthermore, the drug except an allergenic protein from cedar pollen, especially proteins and peptides, can be stably maintained.
In addition, since the dissolution time of the sheet-form preparation of the present invention can be easily controlled by controlling the water content therein, it is suited for intraoral, in particular, sublingual, hyposensitization therapy, which requires control of the sensitization time.
The sheet-form preparation of the present invention may be directly swallowed of course, or may be intraorally dissolved immediately and then swallowed. Further, absorption of the drug through the oral mucous membrane or sublingual mucous membrane can be expected by adjusting the intraoral dissolution time. Since the preparation can be wholly dissolved at a temperature close to the body temperature so that it causes no sense of residues, and since the preparation has a sheet shape so that its surface area is larger than that of tablets or the like and patients and caregivers can easily pinch it, the QOL of the patients and caregivers can be greatly improved.

The sheet-form preparation of the present invention can be produced by, for instance, a method having the steps of: preparing a mixed solution by mixing water, gelatin, and a drug except an allergenic protein from cedar pollen; and forming a thin film using the mixed solution, wherein the water content in the obtained sheet-form preparation is adjusted by adjusting the amount of water to be added in the step of preparing a mixed solution or by drying the thin film after the step of forming the thin film. Such a method for producing the sheet-form preparation of the present invention is also one aspect of the present invention.

In the step of preparing a mixed solution, for instance, gelatin and other additives are first dissolved in a predetermined amount of water at ordinary temperature or under heating, and additives that do not dissolve are dispersed homogeneously to prepare a gelatin solution. If the drug is thermally stable, the drug may be added in this step. In contrast, if thermally unstable, the drug is added after the gelatin solution is cooled down to about ordinary temperature to 35°C, and then stir-mixed.
If foaming occurs during the preparation of a mixed solution, it is adequate to leave the solution overnight or to perform vacuum or reduced pressure degassing.

In the step of forming a thin film, for instance, a predetermined amount of the mixed solution is dispensed into a plastic blister case of a desired size at a temperature of 28°C to 32°C, and cool-solidified immediately after the dispensation to form the thin film. In lieu of the dispensation method, a suitable amount of the mixed solution may be spread over a release film and cool-solidified to form the thin film, and then the film may be cut into a desired size.
The thin film formed in the present step preferably has a size equal to the sheet-form preparation of the present invention descried above.

In the method for producing the sheet-form preparation of the present invention, the water content in the obtained sheet-form preparation is adjusted by adjusting the amount of water to be added in the step of preparing a mixed solution or by drying the thin film after the step of forming a thin film.
That is, in the case of adjusting the water content in the step of preparing a mixed solution by adjusting the amount of water to be added, the sheet-form preparation of the present invention can be produced by forming the thin film described above.
Meanwhile, in the case of adjusting the water content after the step of forming a thin film by drying the thin film, the sheet-form preparation of the present invention can be produced by drying the thin film described above.
Examples of the method for drying the thin film include a method comprising a cold air drying step or a reduced pressure cool drying step.

The method for producing the sheet-form preparation of the present invention is extremely useful in that, for example, the film can be prepared at low temperatures of 35°C or lower, preferably 30°C or lower, with respect to drugs having extremely low thermal stability.
In addition, the obtained sheet-form preparation is preferably seal-packaged if necessary and treated as a product.

Since the sheet-form preparation of the present invention contains gelatin, it gels at ordinary temperature and can be easily intraorally dissolved at a temperature close to the body temperature. In addition, since the dissolution time of the film can be easily controlled by controlling the water content thereof, the sheet-form preparation of the present invention is suited for intraoral, in particular, sublingual, hyposensitization therapy, which requires control of the sensitization time. Further, when the sheet-form preparation of the present invention contains gelatin and a specific additive, the physical properties during use can be significantly improved. The preparation can stably maintain the drug except an allergenic protein from cedar pollen, especially proteins and peptides.
Further, the sheet-form preparation of the present invention may be directly swallowed, or may be immediately intraorally dissolved and then be swallowed. Furthermore, absorption of the drug through the oral mucous membrane or sublingual mucous membrane can be expected by adjusting the intraoral dissolution time. Since the preparation can be wholly dissolved at a temperature close to the body temperature so that it causes no sense of residues, and since the preparation has a sheet shape so that its surface area is larger than that of tablets or the like and patients and caregivers can easily pinch it, the QOL of the patients and caregivers can be greatly improved.
Moreover, the gelatin used in the method for producing the sheet-form preparation of the present invention is one which can be used even in the preparation at lower temperature in comparison with conventional thermoreversible gelling agents. Thus, the sheet-form preparation can be produced while reducing content loss of thermally unstable drugs upon preparation thereof.

The present invention will be described in detail with the following examples; however, the present invention is not limited to these examples.

### (Example 1)

To 74 parts by weight of purified water was added 3 parts by weight of crystalline cellulose A (average particle size: 20 µm), and the cellulose was ultrasound-dissolved and dispersed therein. Added thereto was 10 parts by weight of fish (tilapia)-derived water-soluble gelatin (average molecular weight: approximately 100,000; hydroxyproline amount: approximately 8.6 mol%), and it was dissolved at ordinary temperature to 40°C. Then, 10 parts by weight of D-sorbitol and 3 parts by weight of PEG 4000 were further dissolved therein. The mixture was dispensed into 5-cm² plastic blister cases (Cryomold (square type) No. 3, Sakura Finetek Japan Co., Ltd.) in 1-g portions, and the portions were cool-solidified at 2°C to 8°C for one day and one night. Thereby, sheet-form preparations were obtained.

### (Example 2)

To 74 parts by weight of purified water was added 3 parts by weight of crystalline cellulose A (average particle size: 20 µm), and the cellulose was ultrasound-dissolved and dispersed therein. Added thereto was 10 parts by weight of gelatin (fish-derived) (average molecular weight: approximately 100,000; hydroxyproline amount: approximately 8.6 mol%), and it was dissolved at 50°C to 70°C, and shaken in a shaker under a constant temperature of 40°C. Then, 10 parts by weight of D-sorbitol and 3 parts by weight of PEG 4000 were dissolved into the solution. The mixture was dispensed into 5-cm² plastic blister cases (Cryomold (square type) No. 3, Sakura Finetek Japan Co., Ltd.) in 1-g portions, and the portions were cool-solidified at 2°C to 8°C for one day and one night. Thereby, sheet-form preparations were obtained.

### (Examples 3 and 7)

Sheet-form preparations were obtained in the same manner as in Example 2 except that the compositions were those indicated in Table 1.
In Example 3, gelatin A (pig-derived) (average molecular weight: approximately 85,000; hydroxyproline amount: approximately 9.2 mol%) was used; in Example 4, alkaline-treated gelatin A (pig-derived) (average molecular weight: approximately 180,000; hydroxyproline amount: approximately 9.2 mol%) was used; in Example 5, acid-treated gelatin (pig-derived) (average molecular weight: approximately 100,000; hydroxyproline amount: approximately 9.2 mol%) was used; in Example 6, gelatin B (pig-derived) (average molecular weight: approximately 100,000; hydroxyproline amount: approximately 9.4 mol%) was used; and in Example 7, gelatin (cattle-derived) (average molecular weight: approximately 200,000; hydroxyproline amount: approximately 9.5 mol%) was used.

### (Comparative Example 1)

To 74 parts by weight of purified water was added 3 parts by weight of crystalline cellulose A (average particle size: 20 µm), and the cellulose was ultrasound-dissolved and dispersed therein. Added thereto was 10 parts by weight of agar, and it was dissolved at 80°C to 90°C. Then, 10 parts by weight of D-sorbitol and 3 parts by weight of PEG 4000 were added and mix-dissolved thereinto. The mixture was dispensed into 5-cm² plastic blister cases (Cryomold (square type) No. 3, Sakura Finetek Japan Co., Ltd.) in 1-g portions, and the portions were cool-solidified at 2°C to 8°C for one day and one night. Thereby, sheet-form preparations were obtained.

### (Comparative Examples 2 to 4)

Sheet-form preparations were obtained in the same manner as in Comparative Example 1 except that the compositions were those indicated in Table 1.

**[Table 1]**

| Ingredient name | Examples [parts by weight] | | | | | | | Comparative Examples [parts by weight] | | | |
|---|---|---|---|---|---|---|---|---|---|---|---|
| | 1 | 2 | 3 | 4 | 5 | 6 | 7 | 1 | 2 | 3 | 4 |
| Water-soluble gelatin (fish-derived) | 10 | - | - | - | - | - | - | - | - | - | - |
| Gelatin (fish-derived) | - | 10 | - | - | - | - | - | - | - | - | - |
| Gelatin A (pig-derived) | - | - | 10 | - | - | - | - | - | - | - | - |
| Alkali-treated gelatin (pig-derived) | - | - | - | 10 | - | - | - | - | - | - | - |
| Acid-treated gelatin (pig-derived) | - | - | - | - | 10 | - | - | - | - | - | - |
| Gelatin B (pig-derived) | - | - | - | - | - | 10 | - | - | - | - | - |
| Gelatin (cattle-derived) | - | - | - | - | - | - | 10 | - | - | - | - |
| Agar | - | - | - | - | - | - | - | 10 | - | - | - |
| Carrageenan | - | - | - | - | - | - | - | - | 10 | - | - |
| Locust bean gum | - | - | - | - | - | - | - | - | - | 10 | - |
| Starch | - | - | - | - | - | - | - | - | - | - | 10 |
| D-sorbitol | 10 | 10 | 10 | 10 | 10 | 10 | 10 | 10 | 10 | 10 | 10 |
| PEG 4000 | 3 | 3 | 3 | 3 | 3 | 3 | 3 | 3 | 3 | 3 | 3 |
| Crystalline cellulose A | 3 | 3 | 3 | 3 | 3 | 3 | 3 | 3 | 3 | 3 | 3 |
| Purified water | 74 | 74 | 74 | 74 | 74 | 74 | 74 | 74 | 74 | 74 | 74 |
| Dispensed amount [g/blister] | 1 | 1 | 1 | 1 | 1 | 1 | 1 | 1 | 1 | 1 | 1 |
| Size [cm²] | 5 | 5 | 5 | 5 | 5 | 5 | 5 | 5 | 5 | 5 | 5 |

### (Example 8)

To 80 parts by weight of purified water was added 10 parts by weight of fish (tilapia)-derived water-soluble gelatin (average molecular weight: approximately 100,000; hydroxyproline amount: approximately 8.6 mol%), and the gelatin was dissolved at ordinary temperature to 40°C. Then, 10 parts by weight of D-sorbitol was further added thereto and dissolved therein. The mixture was dispensed into 5-cm² plastic blister cases (Cryomold (square type) No. 3, Sakura Finetek Japan Co., Ltd.) in 1-g portions, and the portions were cool-solidified at 2°C to 8°C for one day and one night. Thereby, sheet-form preparations were obtained.

### (Examples 9 to 14, Comparative Example 5)

Sheet-form preparations were obtained in the same manner as in Example 8 except that the compositions were those indicated in Table 2.

**[Table 2]**

| Ingredient name | Examples [parts by weight] | | | | | | | Comparative Example [parts by weight] |
|---|---|---|---|---|---|---|---|---|
| | 8 | 9 | 10 | 11 | 12 | 13 | 14 | 5 |
| Water-soluble gelatin (fish-derived) | 10 | 10 | 10 | 10 | 10 | 10 | 10 | 10 |
| D-sorbitol | 10 | - | - | - | - | - | - | - |
| Isomalt A | - | 10 | - | - | - | - | - | - |
| Isomalt B | - | - | 10 | - | - | - | - | - |
| Glucose | - | - | - | 10 | - | - | - | - |
| Raffinose | - | - | - | - | 10 | - | - | - |
| D-mannitol | - | - | - | - | - | 10 | - | - |
| Sucralose | - | - | - | - | - | - | 10 | - |
| Purified water | 80 | 80 | 80 | 80 | 80 | 80 | 80 | 80 |
| Dispensed amount [g/blister] | 1 | 1 | 1 | 1 | 1 | 1 | 1 | 1 |
| Size [cm²] | 5 | 5 | 5 | 5 | 5 | 5 | 5 | 5 |

### (Example 15)

To 77 parts by weight of purified water was added 10 parts by weight of fish (tilapia)-derived water-soluble gelatin (average molecular weight: approximately 100,000; hydroxyproline amount: approximately 8.6 mol%), and the gelatin was dissolved at ordinary temperature to 40°C. Then, 10 parts by weight of D-sorbitol and 3 parts by weight of PEG 400 were further added thereto and dissolved therein. The mixture was dispensed into 5-cm² plastic blister cases (Cryomold (square type) No. 3, Sakura Finetek Japan Co., Ltd.) in 1-g portions, and the portions were cool-solidified at 2°C to 8°C for one day and one night. Thereby, sheet-form preparations were obtained.

### (Examples 16 to 20)

Sheet-form preparations were obtained in the same manner as in Example 15 except that the compositions were those indicated in Table 3.

### (Comparative Example 6)

To 80 parts by weight of purified water was added 10 parts by weight of fish (tilapia)-derived water-soluble gelatin (average molecular weight: approximately 100,000; hydroxyproline amount: approximately 8.6 mol%), and the gelatin was dissolved at ordinary temperature to 40°C. Then, 10 parts by weight of D-sorbitol was further added thereto and dissolved therein. The mixture was dispensed into 5-cm² plastic blister cases (Cryomold (square type) No. 3, Sakura Finetek Japan Co., Ltd.) in 1-g portions, and the portions were cool-solidified at 2°C to 8°C for one day and one night. Thereby, sheet-form preparations were obtained.

**[Table 3]**

| Ingredient name | Examples [parts by weight] | | | | | | Comparative Example [parts by weight] |
|---|---|---|---|---|---|---|---|
| | 15 | 16 | 17 | 18 | 19 | 20 | 6 |
| Water-soluble gelatin (fish-derived) | 10 | 10 | 10 | 10 | 10 | 10 | 10 |
| D-sorbitol | 10 | 10 | 10 | 10 | 10 | 10 | 10 |
| PEG 400 | 3 | - | - | - | - | - | - |
| PEG 600 | - | 3 | - | - | - | - | - |
| PEG 2000 | - | - | 3 | - | - | - | - |
| PEG 4000 | - | - | - | 3 | - | - | - |
| PEG 6000 | - | - | - | - | 3 | - | - |
| PEG 20000 | - | - | - | - | - | 3 | - |
| Purified water | 77 | 77 | 77 | 77 | 77 | 77 | 80 |
| Dispensed amount [g/blister] | 1 | 1 | 1 | 1 | 1 | 1 | 1 |
| Size [cm²] | 5 | 5 | 5 | 5 | 5 | 5 | 5 |

### (Example 21)

To 74 parts by weight of purified water was added 3 parts by weight of crystalline cellulose B (average particle size: 50 µm), and the cellulose was ultrasound-dissolved and dispersed therein. Then, 10 parts by weight of fish (tilapia)-derived water-soluble gelatin (average molecular weight: 100,000, hydroxyproline amount: approximately 8.6 mol%) was added thereto and dissolved thereinto at ordinary temperature to 40°C. Further, 10 parts by weight of D-sorbitol and 3 parts by weight of PEG 4000 were added thereto and dissolved thereinto. The mixture was dispensed into 5-cm² plastic blister cases (Cryomold (square type) No. 3, Sakura Finetek Japan Co., Ltd.) in 1-g portions, and the portions were cool-solidified at 2°C to 8°C for one day and one night. Thereby, sheet-form preparations were obtained.

### (Examples 22 to 25)

Sheet-form preparations were obtained in the same manner as in Example 21 except that the compositions were those indicated in Table 4.
In Example 22, crystalline cellulose C (average particle size: 90 µm) was used; in Example 23, powder cellulose A (average particle size: 6 µm) was used; in Example 24, powder cellulose B (average particle size: 10 µm) was used; and in Example 25, powder cellulose C (average particle size: 50 µm) was used.

### (Comparative Example 7)

To 77 parts by weight of purified water was added 10 parts by weight of fish (tilapia)-derived water-soluble gelatin (average molecular weight: approximately 100,000; hydroxyproline amount: approximately 8.6 mol%), and the gelatin was dissolved at ordinary temperature to 40°C. Then, 10 parts by weight of D-sorbitol and 3 parts by weight of PEG 4000 were further added thereto and dissolved therein. The mixture was dispensed into 5-cm² plastic blister cases (Cryomold (square type) No. 3, Sakura Finetek Japan Co., Ltd.) in 1-g portions, and the portions were cool-solidified at 2°C to 8°C for one day and one night. Thereby, sheet-form preparations were obtained.

**[Table 4]**

| Ingredient name | Examples [parts by weight] | | | | | Comparative Example [parts by weight] |
|---|---|---|---|---|---|---|
| | 21 | 22 | 23 | 24 | 25 | 7 |
| Water-soluble gelatin (fish-derived) | 10 | 10 | 10 | 10 | 10 | 10 |
| D-sorbitol | 10 | 10 | 10 | 10 | 10 | 10 |
| PEG 4000 | 3 | 3 | 3 | 3 | 3 | 3 |
| Crystalline cellulose B | 3 | - | - | - | - | - |
| rystalline cellulose C | - | 3 | - | - | - | - |
| Powder cellulose A | - | - | 3 | - | - | - |
| Powder cellulose B | - | - | - | 3 | - | - |
| Powder cellulose C | - | - | - | - | 3 | - |
| Purified water | 74 | 74 | 74 | 74 | 74 | 77 |
| Dispensed amount [g/blister] | 1 | 1 | 1 | 1 | 1 | 1 |
| Size [cm²] | 5 | 5 | 5 | 5 | 5 | 5 |

### (Examples 26 to 29)

Sheet-form preparations were obtained in the same manner as in Example 1 except that the compositions were those indicated in Table 5.
Part of the purified water in the respective sheet-form preparations in Examples 1 to 29 was replaced with the equivalence of a drug except an allergenic protein from cedar pollen. Thereby, sheet-form preparations containing the drug were obtained.

**[Table 5]**

| Ingredient name | Examples [parts by weight] | | | |
|---|---|---|---|---|
| | 26 | 27 | 28 | 29 |
| Water-soluble gelatin (fish-derived) | 50 | 30 | 10 | 5 |
| D-sorbitol | 10 | 10 | 10 | 10 |
| PEG 4000 | 3 | 3 | 3 | 3 |
| Crystalline cellulose A | 3 | 3 | 3 | 3 |
| Purified water | 34 | 54 | 74 | 79 |
| Gelatin/purified water [%] | 147 | 56 | 14 | 6 |
| Dispensed amount [g/blister] | 2 | 2 | 2 | 2 |
| Size [cm²] | 5 | 5 | 5 | 5 |

### (Example 30)

To 66.5 parts by weight of purified water was added 3 parts by weight of crystalline cellulose A (average particle size: 20 µm), and the cellulose was ultrasound-dissolved and dispersed therein. Then, 10 parts by weight of fish (tilapia)-derived water-soluble gelatin was added thereto and dissolved therein at 30°C to 50°C, and shaken in a shaker under a constant temperature of 28°C to 32°C. Further, 0.5 parts by weight of zolmitriptan, 15 parts by weight of D-sorbitol, and 5 parts by weight of PEG 4000 were added thereto and dissolved therein. The mixture was dispensed into 1-cm² plastic blister cases (Cryomold (square type) No. 3, Sakura Finetek Japan Co., Ltd.) in 0.5-g portions, and the portions were cool-solidified at 2°C to 8°C for one day and one night. Thereby, sheet-form preparations were obtained.

### (Example 31)

To 27 parts by weight of purified water was added 3 parts by weight of crystalline cellulose A (average particle size: 20 µm), and the cellulose was ultrasound-dissolved and dispersed therein. Then, 10 parts by weight of fish (tilapia)-derived water-soluble gelatin was added thereto and dissolved therein at 30°C to 50°C, and shaken in a shaker under a constant temperature of 28°C to 32°C to prepare a gelatin solution. Separately, 50 parts by weight of a human insulin 100 U solution was prepared, and 7 parts by weight of D-sorbitol and 3 parts by weight of PEG 4000 were dissolved therein at 2°C to 8°C. This mixture was heated to reach 25°C to 30°C and the whole was added to the gelatin solution prepared beforehand, and they were immediately mixed at 28°C to 32°C. The mixture was dispensed into 5-cm² plastic blister cases (Cryomold (square type) No. 3, Sakura Finetek Japan Co., Ltd.) in 2-g portions, and the portions were cool-solidified at 2°C to 8°C for one day and one night. Thereby, sheet-form preparations were obtained.

### (Example 32)

To 59.8 parts by weight of purified water was added 5 parts by weight of crystalline cellulose A (average particle size: 20 µm), and the cellulose was ultrasound-dissolved and dispersed therein. Then, 20 parts by weight of fish (tilapia)-derived water-soluble gelatin was added thereto and dissolved therein at 30°C to 50°C, and shaken in a shaker under a constant temperature of 30°C to 35°C to prepare a gelatin solution. Further, 0.2 parts by weight of mite antigen DerfI, 10 parts by weight of D-sorbitol, and 5 parts by weight of PEG 4000 were added thereto and immediately mix-dissolved therein at 28°C to 32°C. The mixture was dispensed into 1-cm² plastic blister cases (Cryomold (square type) No. 1, Sakura Finetek Japan Co., Ltd.) in 0.5-g portions, and the portions were cool-solidified at 2°C to 8°C for one day and one night. Thereby, sheet-form preparations were obtained.

### (Example 33)

Sheet-form preparations were obtained in the same manner as in Example 32 except that the compositions were those indicated in Table 6.

**[Table 6]**

| Ingredient name | Examples [parts by weight] | | | |
|---|---|---|---|---|
| | 30 | 31 | 32 | 33 |
| Zolmitriptan | 0.5 | - | - | |
| Human insulin 100 U solution | - | 50 | - | |
| Mite antigen Der f I | - | - | 0.2 | |
| Mite antigen Der f II | | | | 0.2 |
| Water-soluble gelatin (fish-derived) | 10 | 10 | 20 | 20 |
| D-sorbitol | 15 | 7 | 10 | 10 |
| PEG 4000 | 5 | 3 | 5 | 5 |
| Crystalline cellulose A | 3 | 3 | 5 | 5 |
| Purified water | 66.5 | 27 | 59.8 | 59.8 |
| Dispensed amount [g/blister] | 0.5 | 2 | 0.5 | 0.5 |
| Size [cm²] | 1 | 5 | 1 | 1 |

### [Test Methods]

The sheet-form preparations prepared in the respective examples and comparative examples were evaluated on intraoral solubility, gelling temperature during preparation, sensory test (touch), and storage stability at 25°C storage (the sensory test (touch)). Some of the samples were further measured for intraoral dissolution time, intraoral sensory test (roughness), and melting point. The following describes the respective test methods, and Tables 7 to 9 show the results.

### (Intraoral dissolution time measurement)

The test was carried out according to the disintegration test described in the Japanese Pharmacopoeia 15th edition. Distilled water was introduced in a 1000-mL low-form beaker, and the test was carried out at 37 ± 2°C, under conditions where the test container was raised and lowered 29 to 32 times with an amplitude of 53 to 57 mm in one minute. A sheet-form preparation was introduced in the test container, and the test was started under the conditions described above. The time from the beginning of the test to when the sheet-form preparation was completely dissolved and disappeared from the test container was taken as the intraoral dissolution time.

### (Evaluation of intraoral solubility)

A 1000-mL glass Petri dish was charged with 900 mL of phosphate buffer with a pH of 6.8. A stainless-steel sieve (φ 4 mm) was submerged therein upside down, and the solution was stirred using a stirrer (300 rpm). The temperature of this solution was controlled to 37 ± 2°C with a constant temperature water circulator, and a test specimen (5 cm²) was submerged therein. The specimen was left for 10 minutes from the time of submerging the specimen, and whether the specimen was left on the stainless-steel sieve was observed. The evaluation was performed based on the following criteria.
4: No residues present
1: Residues present

### (Storage stability test)

The prepared sheet-form preparations were stored in a thermostatic chamber set to 25°C. The sheet-form preparations were taken out one month after the beginning of the storage, and the sensory test (touch) was carried out. The evaluation method was according to the sensory test (touch).

### (Sensory test (touch))

The sheet-form preparations which were cut in the examples and the comparative examples were actually touched with a finger for five seconds while drawing a circle, and discomfort was evaluated from the viewpoints of whether they were sticky and whether the finger became wet. The evaluation criteria were as follows:
4: the preparation was not sticky and the finger did not become wet;
3: the preparation was slightly sticky or the finger became slightly wet;
2: discomfort was experienced from stickiness and finger wetness; and
1: the preparation was considerably sticky and remained on the finger.

### (Gelling temperature during preparation)

In order to evaluate whether the preparation can be prepared at low temperatures, the temperature at which gelling occurs and the viscosity drastically increases during preparation was evaluated. The evaluation criteria are as follow:
4: lower than 30°C;
3: 30°C or higher, lower than 40°C;
2: 40°C or higher, lower than 50°C; and
1: 50°C or higher.

### (Measurement of melting point)

In order to evaluate whether the sheet-form preparation can be stored at room temperature, the temperature at which each sheet-form preparation melted was measured. The temperature at which the sheet-form preparation started to melt was visually observed and evaluated. The evaluation criteria are as follows:
4: 30°C or higher; and
1: lower than 30°C.

### (Intraoral roughness)

The preparation was actually administered to two subjects, and the subjects dissolved the preparation on the tongue, and evaluated roughness at this time. The evaluation criteria were as follows:
4: no roughness was felt;
3: slight roughness was felt, but no discomfort was felt;
2: roughness was felt, but no residues were felt;
1: significant roughness was felt, and residues were felt.

**[Table 7]**

| Examples | In preparation | | Intraoral solubility | 25°C 1 month storage | Total |
|---|---|---|---|---|---|
| | Gelling temperature in preparation | Sense (touch) | | Sense (touch) | |
| Example 1 | 4 | 4 | 4 | 4 | 16 |
| Example 2 | 4 | 4 | 4 | 4 | 16 |
| Example 3 | 4 | 4 | 4 | 4 | 16 |
| Example 4 | 3 | 4 | 4 | 4 | 15 |
| Example 5 | 3 | 4 | 4 | 4 | 15 |
| Example 6 | 3 | 4 | 4 | 4 | 15 |
| Example 7 | 3 | 4 | 4 | 4 | 15 |
| Comparative Example 1 | 1 | 4 | 1 | 4 | 10 |
| Comparative Example 2 | 2 | 3 | 1 | 3 | 9 |
| Comparative Example 3 | 2 | 3 | 1 | 3 | 9 |
| Comparative Example 4 | 2 | 1 | 4 | 1 | 8 |
| Example 8 | 4 | 4 | 4 | 3 | 15 |
| Example 9 | 4 | 4 | 4 | 3 | 15 |
| Example 10 | 4 | 4 | 4 | 3 | 15 |
| Example 11 | 4 | 4 | 4 | 3 | 15 |
| Example 12 | 4 | 4 | 4 | 3 | 15 |
| Example 13 | 4 | 4 | 4 | 3 | 15 |
| Example 14 | 4 | 4 | 4 | 3 | 15 |
| Comparative Example 5 | 4 | 2 | 4 | 2 | 12 |
| Example 15 | 4 | 3 | 4 | 3 | 14 |
| Example 16 | 4 | 3 | 4 | 3 | 14 |
| Example 17 | 4 | 4 | 4 | 4 | 16 |
| Example 18 | 4 | 4 | 4 | 4 | 16 |
| Example 19 | 4 | 4 | 4 | 4 | 16 |
| Example 20 | 4 | 3 | 4 | 3 | 14 |
| Comparative Example 6 | 4 | 2 | 4 | 2 | 12 |

**[Table 8]**

| Examples | In preparation | | Intraoral solubility | Melting point | Intraoral sense (roughness) | Total |
|---|---|---|---|---|---|---|
| | Gelling temperature in preparation | Sense (touch) | | | | |
| Example 21 | 4 | 4 | 4 | 4 | 4 | 20 |
| Example 22 | 4 | 4 | 4 | 4 | 3 | 19 |
| Example 23 | 4 | 4 | 4 | 4 | 2 | 18 |
| Example 24 | 4 | 4 | 4 | 4 | 4 | 20 |
| Example 25 | 4 | 4 | 4 | 4 | 3 | 19 |
| Comparative Example 7 | 4 | 4 | 4 | 1 | 4 | 17 |

**[Table 9]**

| Examples | Gelatin/purified water [wt%] | Intraoral dissolution time [sec] |
|---|---|---|
| 26 | 147 | 1385 |
| 27 | 56 | 68 |
| 28 | 14 | 24 |
| 29 | 6 | 13 |
| 30 | 15 | 37 |
| 31 | 16* | 25 |
| 32 | 33 | 39 |
| 33 | 33 | 42 |

| | | |
|---|---|---|
| *: Water is included in the human insulin 100 U solution, and the amount thereof was also included in calculation. | | |

As shown in Tables 7 to 9, the sheet-form preparations of the examples each showed good results in the respective evaluation items, whereas the sheet-form preparations of the comparative examples showed no good result in the respective evaluation items.

Since the sheet-form preparation of the present invention contains gelatin, it gels at ordinary temperature and can be easily dissolved at a temperature close to the intraoral body temperature. In addition, since the dissolution time of the film can be easily controlled by controlling the water content thereof, the sheet-form preparation of the present invention is suited for intraoral, in particular sublingual, hyposensitization therapy, which requires control of the sensitization time. Further, when the sheet-form preparation of the present invention contains gelatin and a specific additive, the physical properties during use can be significantly improved. The preparation can stably maintain the drug except an allergenic protein from cedar pollen, especially proteins and peptides.
Further, the sheet-form preparation of the present invention may be directly swallowed, or may be immediately intraorally dissolved and swallowed. Furthermore, absorption of the drug through the oral mucous membrane or sublingual mucous membrane can be expected by adjusting the intraoral dissolution time. Since the preparation can be wholly dissolved at a temperature close to the body temperature so that it causes no sense of residues, and since the preparation has a sheet shape so that its surface area is larger than that of tablets or the like and patients and caregivers can easily pinch it, the QOL of the patients and caregivers can be greatly improved.
Moreover, in the method for producing the sheet-form preparation of the present invention, gelatin to be used can be stable even in preparation at lower temperatures in comparison with conventional tharmoreversible gelling agents. Thus, the sheet-form preparation can be produced while reducing content loss of thermally unstable drugs upon preparation thereof.

## Claims

1. A sheet-form preparation comprising:
water;
gelatin; and
a drug except an allergenic protein from cedar pollen.

2. The sheet-form preparation according to claim 1, further comprising at least one selected from the group consisting of sugars, sugar alcohols, and sugar fatty acids.

3. The sheet-form preparation according to claim 1 or 2, further comprising polyethylene glycol or a derivative thereof.

4. The sheet-form preparation according to claim 1, 2, or 3, further comprising crystalline cellulose.

5. The sheet-form preparation according to claim 1, 2, 3, or 4,
wherein the drug except an allergenic protein from cedar pollen is provided in a liquid or solid form containing this drug.

6. The sheet-form preparation according to claim 1, 2, 3, 4, or 5,
wherein the amount of the gelatin is 2 to 40 wt% based on the total weight.

7. The sheet-form preparation according to claim 1, 2, 3, 4, 5, or 6, which has a thickness within the range of 30 to 5000 µm.

8. The sheet-form preparation according to claim 1, 2, 3, 4, 5, 6, or 7, which has a planar surface area within the range of 0.5 to 6.0 cm².

9. A method for producing the sheet-form preparation according to claim 1, the method comprising the steps of:
preparing a mixed solution by mixing water, gelatin, and a drug except an allergenic protein from cedar pollen; and
forming a thin film using the mixed solution,
wherein a water content in the obtained sheet-form preparation is adjusted by adjusting the amount of water to be added in the step of preparing a mixed solution or by drying the thin film after the step of forming a thin film.
